# EUROPEAN PATENT APPLICATION

(11) **EP 1 479 759 A2**
(43) Date of publication of application: **24.11.2004**
(21) Application number: 04008321.4
(22) Date of filing: 06.04.2004
(51) Int. Cl.: C12M 3/00, C12N 15/89

(54) **System and apparatus for injecting substance into cell**

(30) Priority: 21.05.2003 JP 2003142970
(71) Applicant: FUJITSU LIMITED, Kawasaki-shi, Kanagawa 211-8588 (JP)
(72) Inventor: Sasaki, Jun, Kawasaki-shi Kanagawa 211-8588 (JP); Youoku, Sachihiro, Kawasaki-shi Kanagawa 211-8588 (JP); Tamamushi, Kazuo, Kawasaki-shi Kanagawa 211-8588 (JP); Ito, Akio, Kawasaki-shi Kanagawa 211-8588 (JP)
(74) Representative: HOFFMANN - EITLE

(57) **Abstract**

An apparatus for injecting a substance into a cell includes a cell input part to input the cell, a cell output part to output the cell, a flow path that connects the cell input part and the cell output part, wherein the cell flows in the flow path from the cell input part toward the cell output part, a trapping part that is arranged in the flow path and traps the cell that flows in the flow path, and an inserting part that allows to pass a needle that injects the substance into the cell that is trapped at the trapping part. The cell injected with the substance flows in the flow path toward the cell output part and the cell can be retrieved from the cell output part.

## Description

### BACKGROUND OF THE INVENTION

### 1) Field of the Invention

The present invention relates to a system and an apparatus for injecting a substance, such as a gene solution or a drug solution, into a cell.

### 2) Description of the Related Art

If is common to use gene-injected cells or the drug-injected cells in the field of life science such as the regenerative medicine and the genome-based drug discovery. Such cells are used for conducting pure research or are used in the field of medicine. Various gene-transferring technologies have been used to produce such cells.

However, the technologies used to produce such cells for the purpose of the research can not be applied to produce such cells used in the field of the medicine; because, the requirements in the field of the medicine are different from those for the purposes of the research. The requirements in the field of the medicine include:
1) possibility of use of any type of cells or substances to be transferred (hereinafter, "transfer substance"),
2) high accuracy, and
3) possibility of mass production.

It is difficult for the conventional technologies to satisfy the requirement "possibility of use of any type of cells or transfer substance". In other words, the combinations of the cells and the transfer substances that can be used in most of the conventional technologies are limited. This does not create much problem in the case of the research; because, in the case of the research, only those combinations that can be used in the available conventional technologies are used. However, in the field of the medicine, the combinations of the cells and the transfer substances that are used are fixed. Therefore, in the field of the medicine, a gene-transferring method that does not limit the combinations of the cells and the transfer substances is required.

In the case of the research, many cells are put in suspension and the genes are collectively transferred into the cells by injecting virus vectors or drug solutions into the cells. However, in the case of the medical care, because an individual cell needs to be managed by assigning numbers after injection of a gene in the cell, and because, the individual cell is observed to check whether the effect of the injection appears, the individual cell is required to be handled separately right from the stage of the injection of the gene. Therefore, the method of collectively handling the cells is not suitable for the field of the medicine.

In the case of the regenerative medicine, a vast number of target cells, about 10⁵ to 10⁶, are required at a time. Thus, in the case of the medicine, a method that allows mass production of the cells is required, moreover, the throughput can not be sacrificed for the requirements of the possibility of use of any type of cells or transfer substance and the possibility of handling individual cells.

Conventionally, the genes have been transferred into the cells using methods such as the vector method, the transfection method, the electroporation method, the particle gun method, and the injection method. The vector method is a biological method and the transfection method is a chemical method. The electroporation method, the particle gun method, and the injection method are physical methods.

The biological method and the chemical method are widely used in the research of the molecular biology, but are not suitable for the regenerative medicine; because, these methods limit the combinations of the cells and the transfer substances.

On the other hand, the electroporation method and the particle gun method, which are the physical methods, do not limit the combinations of cells and transfer substances. In the electroportion method, a gene is injected from a hole made by rupturing a cell membrane using an electrical pulse (see Japanese Patent Application Laid-Open Nos. H11-18770 and H11-506630). In the particle gun method, a cell membrane is ruptured by accelerating a microscopic particle, to which a gene is adhered, and shooting a cell with the microscopic particle (see Japanese Patent Application Laid-Open Nos. H6-62871 and H9-248183).

However, in the method of rupturing the cell membrane using an electrical pulse, because the control of the apparatus is difficult, sometimes the cell membrane dies due to rupturing. In the method of shooting the cell with the microscopic particle, because the control of the apparatus is difficult, some of the cells are not properly shoot with the microscopic particles and are left behind with their cell membranes intact. As a result, both the methods have a transfer success-rate of only a few percent.

On the other hand, the injection method (see Japanese Patent Application Laid-Open Nos. H5-192171 and H6-343478), which is widely used for the artificial insemination, has nearly 100% success rate. In the artificial insemination, a skilled worker transfers a sperm into a cell while looking through a microscope or by using a high-resolution controller. Because the worker is skilled and he/she uses a microscope or a high-resolution controller, he/she can control the needle accurately, so that there is not much damage to the cells. Moreover, the injection method does not limit the combinations of the cells and the transfer substances, and has a high success-rate; therefore, this method has been considered to be the most effective method.

However, the injection method has a low throughput. Because, the transfer is carried out by hand while observing the cell put on a petri dish through a microscope, even a skilled worker can work on maximum a several hundred cells per hour. Moreover, the worker has to carry the petri dish that contains the gene-transferred cell to the cultivation apparatus to culture the cell.

Several suggestions have been made to overcome this disadvantage. For example, Japanese Patent Application Laid-Open No. 2000-23657 discloses a method in which the cells are arranged in a one-dimensional array or a two-dimensional array and the genes are injected at a time using capillaries that are arranged in an array.

Japanese Patent Application Laid-Open No. 2002-027969 discloses a method in which the gene is injected while observing each target cell through a microscope. Concretely, the cells are trapped at trapping parts that are arranged in a one-dimensional array on the surface of a silicon substrate, and genes are transferred by moving capillaries that are arranged in an array, while observing the operation through the microscope.

However, the throughput is low in the method disclosed in Japanese Patent Application Laid-Open No. 2000-23657; because, the worker still has to carry the cell to the cultivation apparatus. Moreover, because the cells are arranged at random on the petri dish, it is difficult to handle each cell separately. Moreover, because the cells are of various sizes and shapes, when the capillaries penetrate the cells, some of the capillaries fail to penetrate the cells or even destroy the cells. Thus, this method is not suitable for use in the field of the medicine.

The throughput is also low in the method disclosed in Japanese Patent Application Laid-Open No. 2002-027969; because, the method is not intend for automation of providing and taking off cells. Thus, the method is not suitable for use in the medical care.

### SUMMARY OF THE INVENTION

The object of the present invention is to provide a system and an apparatus, for injecting a substance into a cell, that is suitable for use in the field of the medicine.

An apparatus for injecting a substance into a cell according to an aspect of the present invention includes a cell input part to input the cell; a cell output part to output the cell; a flow path that connects the cell input part and the cell output part, wherein the cell flows in the flow path from the cell input part toward the cell output part; a trapping part that is arranged in the flow path and traps the cell that flows in the flow path; and an inserting part that allows to pass a needle that injects the substance into the cell that is trapped at the trapping part. The cell injected with the substance flows in the flow path toward the cell output part and the cell can be retrieved from the cell output part.

A system for injecting a substance into a cell according to another aspect of the present invention includes the apparatus according to the present invention; a liquid feeder that is connected to the cell input part; a cultivation apparatus that is connected to the cell output part; an apparatus for trapping a cell that is connected to the trapping part; a needle driver that drives the needle; and an observing part and a light source, whereby an operator can observe the cell trapped at the trapping part.

A system for injecting a substance into a cell according to still another aspect of the present invention includes the apparatus according to the present invention; a first liquid feeder that feeds a liquid that contains the cell to the cell input part; a second liquid feeder that retrieves the cell from the cell output part; and a dispenser that receives the cell retrieved by the second liquid feeder.

The other objects, features, and advantages of the present invention are specifically set forth in or will become apparent from the following detailed description of the invention when read in conjunction with the accompanying drawings.

### BRIEF DESCRIPTION OF THE DRAWINGS

Fig. 1 is a schematic of an apparatus according to the present invention;
Fig. 2 is a schematic of a system that includes the apparatus according to a first embodiment of the present invention;
Fig. 3 is an enlarged view of a portion of the system shown in Fig. 2;
Fig. 4 is a cross section of the apparatus near a trapping part;
Fig. 5 is a top plan view of the apparatus near the trapping part;
Fig. 6 is a top plan view of a trapping part of an apparatus according to a second embodiment of the present invention;
Fig. 7 is a cross section of a trapping part of an apparatus according to a third embodiment of the present invention;
Fig. 8 is a schematic diagram of a system according to a fourth embodiment of the present invention;
Fig. 9 is an illustration for explaining how a cell is trapped using suction;
Fig. 10 is a flowchart of a process procedure for producing a gene injected cell according to the present invention;
Fig. 11 is an illustration for explaining how a cell can be trapped using a trapping device; and
Fig. 12 is an illustration for explaining how a cell can be trapped using a cytophilic substance.

### DETAILED DESCRIPTION

Exemplary embodiments of a system and an apparatus for injecting a substance into a cell are explained in detail below with reference to accompanying drawings.

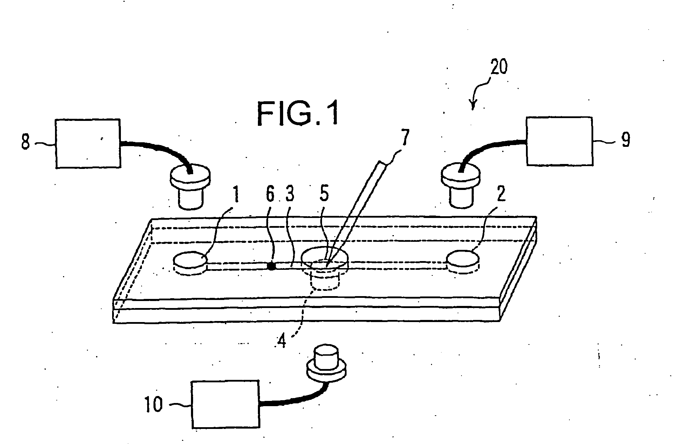

Fig. 1 is a schematic of an apparatus according to the present invention. In the present invention, the cells 6 that are to be subjected to the gene injection or the drug injection are automatically supplied and the cells 6 that are subjected to the gene injection or the drug injection are automatically retrieved so that it is possible to greatly improve the throughput.

The apparatus 20 according to the present invention has a trapping part 4 and an inserting part 5. At least one of the trapping part 4 and the inserting part 5 is formed of a transparent material. Accordingly, the injection operation can be performed while observing the process of injecting the gene solution or the drug solution into the cell 6, which is trapped at the trapping part 4, using a needle 7 that is inserted from the inserting part 5.

The apparatus 20 has a cell input part 1 and a cell output part 2. The apparatus 20 is detachable from the system according to the present invention. Accordingly, the apparatus can be replaced or dismantled easily for the maintenance.

The trapping part 4 is formed in a surface of a flow path 3, and the trapping part 4 has a trapping hole that has a diameter which is sufficiently smaller than the diameter of the cell 6. The trapping hole is used to suck the cell 6. Accordingly, after it is confirmed that the cell 6 comes close to the trapping part 4, the cell 6 can be trapped at the trapping hole by operating an apparatus 10 for trapping that is connected to the trapping part 4.

Alternatively, the apparatus 10 may trap the cell 6 by sucking the cell 6 from the opening that is arranged in the surface of the flow path, or by physically holding the cell 6 using a trapping device that is inserted into the flow path 3 from the opening that is arranged in the surface of the flow path 3.

Alternatively, a cytophilic substance may be provided on the surface of the flow path 3 and the cell 6 may be trapped by using an adhesive force of the cytophilic substance.

Accordingly, the cell 6 that is flowing in the flow path 3 can be trapped easily and individual cell can be operated separately.

Around the trapping part 4, preferably, the flow path 3 is widened, or meandering, or has a step. Accordingly, the traveling speed of the cell 6 slows down around the trapping part 4 so that the cell 6 can be trapped surely in the trapping hole.

The inserting part 5 is positioned above the trapping part 4. The inserting part 5 has an inserting hole of a diameter that is large enough so as not to hinder the insertion of the needle 7. The inserting hole is configured to include where the cell is trapped projectively. In other words, a line that passes through the center of the inserting hole also passes through the center of the cell 6. Accordingly, the injection operation can be performed simply by moving the needle 7 along the line that passes through the center of the inserting hole.

The system according to the present invention includes the apparatus 20 according to the present invention. The system also includes a liquid feeder 8, which is connected to the cell input part 1, a cultivation apparatus 9, which is connected to the cell output part 2, a needle driver (not shown), which is connected to the inserting part 5, and an observing part (not shown) and a light source (not shown), which are used to observe the trapping part 4 and the inserting part 5. The system also includes the apparatus 10, as a suction apparatus, that is connected to the trapping part 4.

The observing part includes a lens unit (not shown), a stage (not shown), and a limb (not shown). The observing part is elevated to a certain distance from the trapping part 4. Specifically, the lens unit is elevated to such a distance that the lens unit does not interfere with the needle driving stroke of the needle driver. For example, the lens unit is elevated by at least 30 millimeter (mm) above the inserting part 5. The apparatus 20 is fixed to the stage. The limb is also fixed perpendicularly to the stage and holds the lens unit in such a manner that the lens unit can rotate around the inserting part 5 at a constant distance from the trapping part 4. Accordingly, the cell 6 trapped at trapping part 4 and the needle 7 inserted from the inserting part 5 can be observed simultaneously from various angles through the lens unit.

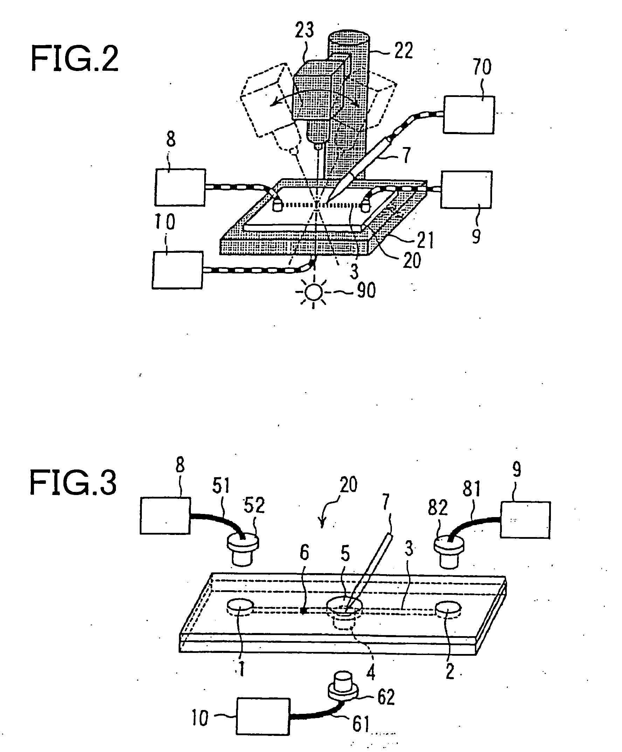

Fig. 2 is a schematic of the system according to a first embodiment of the present invention. Fig. 3 is an enlarged view of a portion of the system according to the first embodiment.

The system includes the apparatus 20, a stage 21, a limb 22, the liquid feeder 8, the apparatus 10, the needle (which is a microneedle) 7, the cultivation apparatus 9, and a light source 90.

The apparatus 20 is fixed to the stage 21. The limb 22 is fixed to the stage 21 and supports a lens unit 23. The liquid feeder 8 transfers carrier liquid and cells 6 to the cell input part 1. The apparatus 10 traps a cell at the trapping part 4 by sucking the cell that is flowing in the flow path 3. The needle 7 transfers the gene solution or the drug solution into the cell that is trapped at the trapping part 4. A needle driver 70 drives the needle 7. The cultivation apparatus 9 takes off the cell in which the gene solution or the drug solution has been transferred, and cultures the cell. The light source 90 illuminates the trapping part 4.

The lens unit 23 is elevated from the trapping part 4 to such a distance that the lens unit 23 does not interfere with a needle driving stroke of the needle driver 70. For example, the lens unit 23 is elevated by preferably 30 mm, and more preferably 40 mm to 50 mm. By elevating the lens unit 23 high, the operation of trapping the cell at the trapping part 4 and the operation of transferring the gene or the drug using the needle 7 can be observed without interfering with the operations.

The limb 22 holds the lens unit 23 in such a manner that the lens unit 23 can be rotated around the inserting part 5 at a constant distance from the inserting part 5. Consequently, the cell trapped at the trapping part 4 can be observed from different angles and the gene transfer can be performed conveniently.

As shown in Fig. 3, the cell input part 1 is arranged on an end of the flow path 3. A detachable component 52 is arranged on an end of a tube 51 that comes from the liquid feeder 8. The detachable component 52 has a screw construction, so that the detachable component 52 can be detachably connected to the cell input part 1.

The cell output part 2 is arranged on another end of the flow path 3. A detachable component 82 is arranged on an end of a tube 81 that comes from the cultivation apparatus 9. The detachable component 82 has a screw construction, so that the detachable component 82 can be detachably connected to the cell output part 2.

The trapping part 4 is arranged almost at the center of the flow path 3 and has an opening. A detachable component 62 is arranged on an end of a tube 61 that comes from the apparatus 10. The detachable component 62 has a screw construction, so that the detachable component 62 can be detachably connected to the opening of the trapping part 4. The apparatus 10 generates a suction to suck the carrier liquid on the flow path 3 and traps a cell 6 at the trapping part 4.

The inserting part 5 is arranged above the trapping part 4 and has an opening. The injection of the gene solution or the drug solution is performed by inserting the needle 7 from the inserting part 5 and penetrating the cell 6, which is trapped at the trapping part 4, with the needle 7.

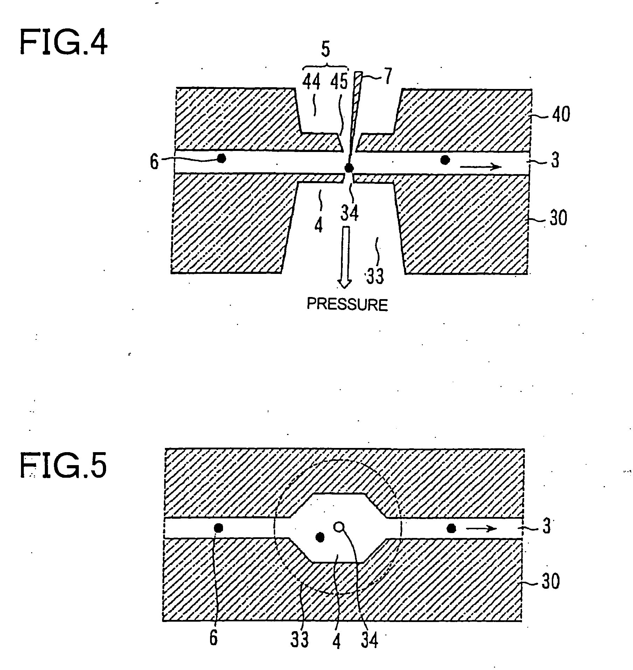

Fig. 4 is a cross-section of the apparatus 20 near the trapping part 4. Fig. 5 is a top plan view of the apparatus 20 near the trapping part 4.

The apparatus 20 includes a bottom plate 30 and an upper plate 40 that are formed of transparent polycarbonate. The flow path 3 is formed in the bottom plate 30 and has a width of 50 micrometers (µm) and a depth of 50 µm. The trapping part 4 is formed at almost the center of the flow path 3. The flow path 3 is made wider near the trapping part 4 so that the flow of the carrier liquid slows down when it comes near the trapping part 4 and it becomes possible to surely and easily trap the cell 6. The width of the flow path 3 near the trapping part 4 is about 150 µm. The width and the depth of the flow path 3 are decided based on the external diameter of the cell 6, which is about 15 µm to 20 µm.

An opening 33, of a diameter 0.5 mm, and a trapping hole 34, of a diameter 5 µm, are formed in the bottom of the flow path 3, by excimer laser processing. The diameter of 5 µm is appropriate to certainly trap the cell 6 at the trapping hole 34 so that the cell 6 does not escape from the trapping hole 34. The apparatus 10 sucks the carrier liquid via the detachable component 62 that is attached to the opening 33, so that the cell 6 is trapped at the trapping hole 34.

In the upper plate 40, the inserting part 5 that is used to insert the needle 7 is arranged at the opposite part of the trapping part 4. The inserting part 5 has an opening 44 and an inserting hole 45 that is arranged in the opening 44. The inserting hole 45 is formed in the upper plate 40 by excimer laser processing. A diameter of the inserting hole 45 is, for example, between 10 µm and 20 µm. The diameter of 10 µm to 20 µm is appropriate to insert the needle 7, which has a diameter of about 1 µm, through the inserting hole 45 while preventing the carrier liquid to spout from the inserting hole 45. The cell input part 1 and the cell output part 2 are formed in the upper plate 40.

Returning to Fig. 3, the cell suspension is delivered from the liquid feeder 8, which uses a liquid chromatography pump or the like, to the cell input part 1. The cell suspension flows toward the trapping part 4 via the flow path 3. The cell suspension is the carrier liquid that includes the cells, that is, the cell suspension is a mixture of the cells and the liquid culture medium.

When the cell 6 enters the flow path 3 and comes close to the trapping part 4, the flowing speed of the cell 6 drops because the flow path 3 is wider around the trapping part 4 (see Fig. 5). An image processing apparatus (not shown) detects the cell 6, via the lens unit 23, in the trapping part 4, and then the apparatus 10 operates to suck the cell and the carrier liquid. As a result, the cell 6 is trapped at the trapping hole 34. The apparatus 20 is formed of transparent material; therefore, whether the cell 6 is trapped can be observed well because the light source 90 illuminates the cell 6.

When the image processing apparatus detects the cell 6 that is trapped at the trapping hole 34, a command is sent to the needle driver 70 via a needle controller (not shown) to move the needle 7 toward the cell 6. The needle 7 passes through the inserting hole 45 and penetrates the cell 6, so that the gene solution or the drug solution in the needle 7 is injected into the cell 6.

The center of the cell 6 that is trapped is on a line that passes through the center of the inserting hole 45; therefore, the needle 7 penetrates the cell 6 when the needle 7 is moved straight downward.

The lens unit 23 is elevated above the upper plate 40 by at least 30 mm; therefore, the lens unit 23 does not interfere with the movement of the needle 7 and the like.

The image processing apparatus confirms whether the injection is completed. When the injection operation is completed, the apparatus 10 operates to release the cell 6 from the trapping hole 34.

The cell 6 released from the trapping part 4 and the carrier liquid enters the narrower part of the flow path 3 and reaches the cell output part 2. The cell 6 is then transferred to the cultivation apparatus 9 via the detachable component 82 that is connected to the cell output part 2 and the tube 81.

The depth and the width of the flow path 3 is about 50 µm; therefore, the cell 6 is trapped almost completely. Even if the cell 6 is not trapped, and if the cultivation apparatus 9 receives the cell 6 into which the gene solution or the drug solution are not injected, no problem occurs because only those cells that show the effect of the injection are picked up finally.

Thus, in the system according to the present invention, all the processes are automated. Moreover, the cell 6 flows faster except near the trapping part 3, so that the throughput is high. Moreover, because the apparatus 20 can be made form a resin material, mass-production of the apparatus 20 is possible at a comparatively low price.

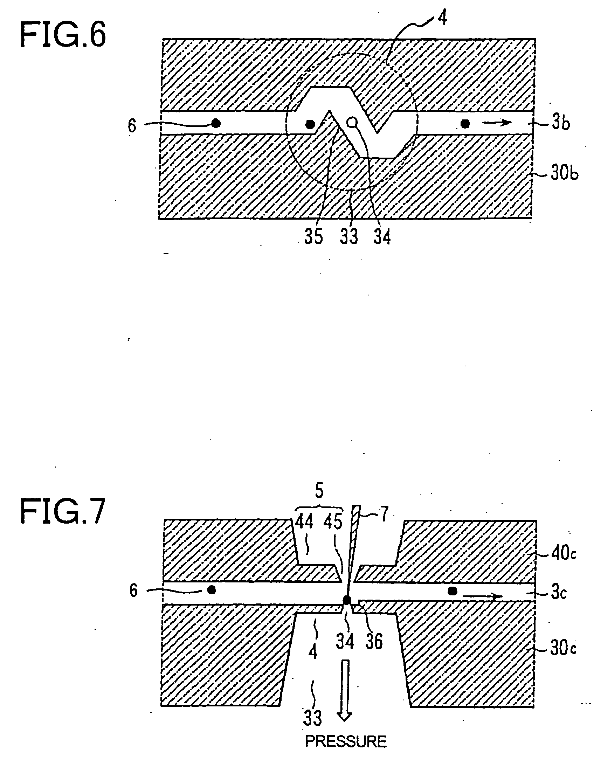

Fig. 6 is a top plan view of a trapping part of an apparatus according to a second embodiment of the present invention. A flow path 3b is formed in a bottom plate 30b. The flow path 3b has a substantially constant width, which is about 50 µm, and has a meandering part 35 around the trapping part 4.

The direction of the flow of the cell suspension changes when the cell suspension is flowing in the meandering part 35, so that the cell suspension slows down and it becomes easier to trap the cell 6.

Fig. 7 is a cross section of a trapping part of an apparatus according to a third embodiment of the present invention. A flow path 3c is formed in a bottom plate 30c. The flow path 3c has a substantially constant width, which is about 50 µm. A step 36 is formed in the flow path 3c, near the trapping part 4, in the downstream side of the trapping part 4. The step 36 blocks the cell 6 so that it becomes easy to trap the cell 6.

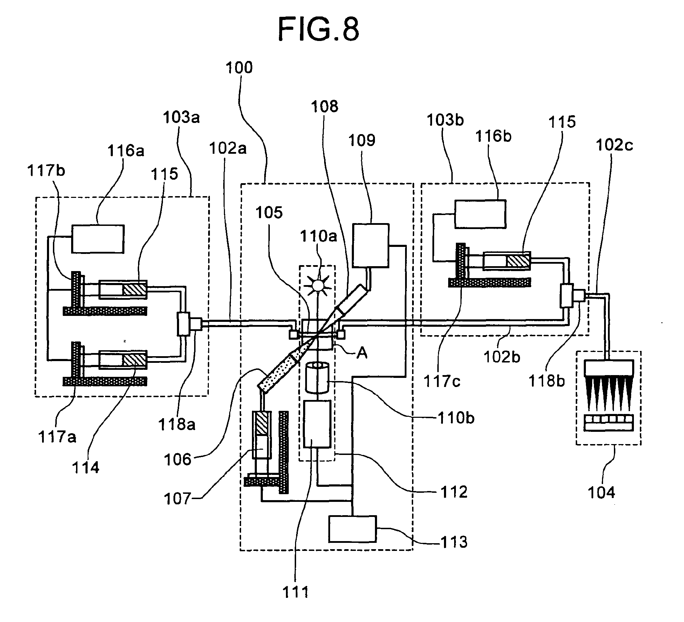

Fig. 8 is a schematic diagram of a system according to a fourth embodiment of the present invention. In the system according to the fourth embodiment, peripherals are added in the previous stage to an apparatus 100 for injecting a substance into a cell and in the subsequent stage to the apparatus 100.

A first liquid feeder 1 03a, which is added in the previous stage, includes a first feeder 117a, which feeds cell suspension 114, a second feeder 117b, which delivers culture medium 115, a feeder controller 116a, and a mixing unit 118a.

The cell suspension 114, which fills the first feeder 117a, and the culture medium 115, which fills the second feeder 117b, are mixed in the mixing unit 118a, then, diluted to the appropriate concentration to inject the transfer substance into the cell separately, and delivered to the apparatus 100 via a pipe 102a.

The feeder controller 116a controls the measure of the feeding liquid and the liquid pressure of both the cell suspension 114 and the culture medium 115, so that the concentration of the cell suspension that is output from the mixing unit 118a is controlled.

The concentration of the cell suspension that fills the first feeder 117a may be about 10⁶ cells/mL. Generally, the concentration of the cell suspension becomes this value after the subculture.

When this cell suspension is diluted in the mixing unit 118a to have one hundredth concentration, the cell suspension with the concentration of about 10⁴ cells/mL is gained. Any known micro liquid feeder for liquid chromatograph may be used in the first feeder 117a, the second feeder 117b, and the feeder controller 116a.

In preferable example, when the amount of the feeding liquid is about 1 µl/min, the processing capacity of the apparatus 100 is estimated to be 10 cells/min based on the concentration of this cell suspension.

The apparatus 100 includes the pipe 102a, a flow path 105, which is connected to a pipe 1 02b that delivers the transfer-substance injected cells, an apparatus 112 for observing a cell, which includes an image processing apparatus that has a light source 110a and an objective lens 110, an apparatus (a trapping part) 106 for trapping a cell, which traps the cells that sequentially flow on the flow path 105 one-by-one, and a needle 108, which punctures the cell membrane of the cell that is trapped to inject a transfer substance. The apparatus 112 observes the flow path 105 constantly, transmits the cell-detection signal, and detects whether the apparatus 106 completes the trapping of the cell.

Moreover, the apparatus 100 includes a controller 107, which controls the apparatus 106, a needle controller 109, which controls the needle 108, and a host computer 113, which controls the controller 107, the needle controller 109, and the apparatus 112.

When the host computer 113 receives the cell-detection signal from the apparatus 112, the host computer 113 sends a trapping start command, to start to trap a cell, to the controller 107. Then when the host computer 113 receives the cell-trap competition signal from the apparatus 112, the host computer 113 commands the needle controller 109 to start the gene injection. After the transfer substance is injected into the cell, the cell is delivered to the second liquid feeder 103b via the flow path 105 and the pipe 102b.

In the apparatus 100, the flow path 105 preferably has the internal diameter that allows the cells to flow in line. In consideration of that the external diameter of the cell is about 15 µm to 20 µm, the internal diameter of the flow path 105 is preferably 50 µm to 100 µm.

The flow path 105 is arranged in the microscope field, which is between the light source 110a and the objective lens 110b, and formed of a transparent material to observe the cell on the flow path 105.

The image processing apparatus 111 detects the states of the cell, the apparatus 106, and the needle 108 that are in the field of view of the microscope, such as whether the cell enters the flow path 105, whether the cell is trapped, whether the gene is injected into the cell.

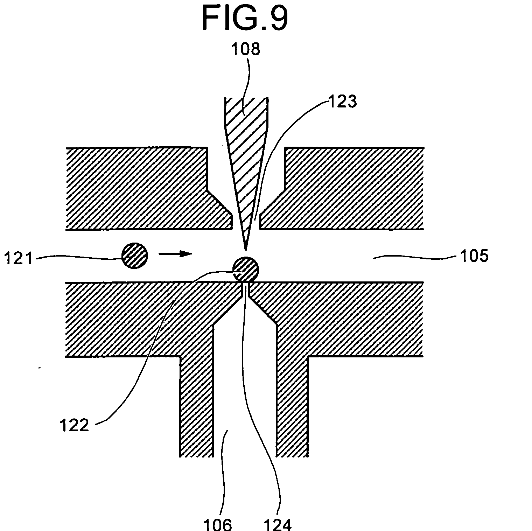

The apparatus 106 may have a configuration shown in Fig. 9. Fig. 9 is an enlarged view of an A part in Fig. 8. The apparatus 106 sucks the liquid and a cell 121 that are in the flow path 105, so that the cell 121 is trapped at a trapping hole 124.

On the other hand, the cell may be trapped mechanically by connecting a trapping device that is driven by the piezoelectric element or the like, or by using the cytophilic substance.

The tip of the needle 108 is required to be sharp enough to penetrate the cell. In other words, the diameter of the tip of the needle 108 has to be preferably about 1 µm. The diameter of an inserting hole 123, which is a micro hole that the needle 108 that is micro runs though, is preferably about 10 µm to 20 µm.

Returning to Fig. 8, a second liquid feeder 103b mixes the culture medium 115 and the cell suspension that includes the transfer-substance injected cells that is delivered via a pipe 1 02b. The second liquid feeder 103b includes a feeder 117c, which feeds the culture medium 115, a mixing unit 118b, which mixes the cell suspension and the culture medium, and a liquid feeder controller 116b, which controls the feeder 117c.

The cell suspension that includes the transfer-substance injected cells is diluted to an appropriate concentration so that individual cells can be treated separately, and then, delivered to a dispenser 104 via a pipe 102c. The dispenser 104 includes a general-purpose multi-hole plate. Any known dispenser may be used as the dispenser 104.

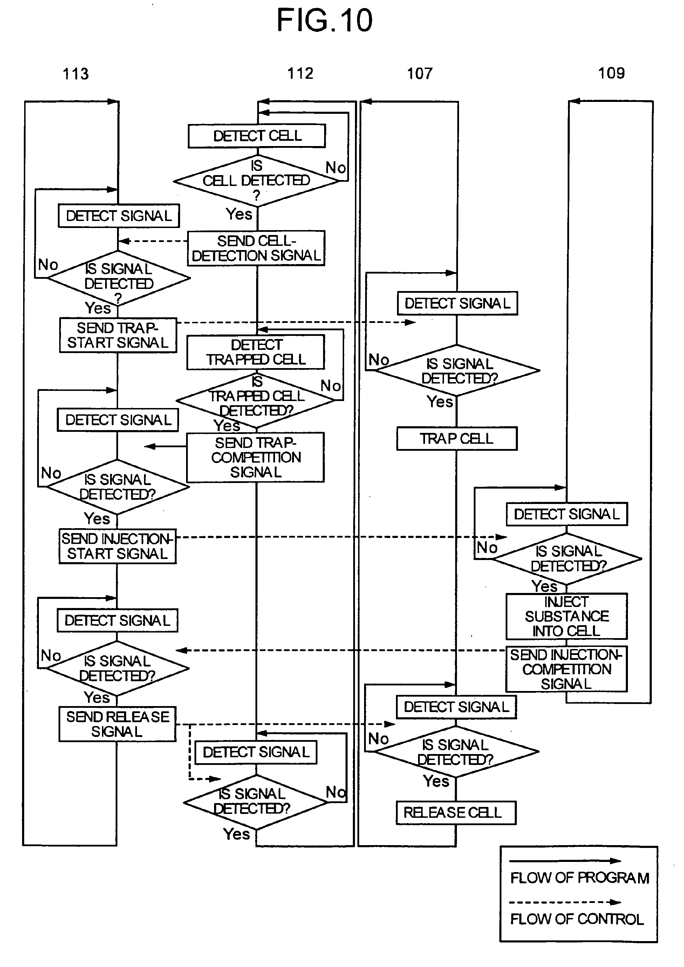

Fig. 10 is a flowchart of operations for producing a gene-injected cell, and the operations of the host computer 113, the image processing apparatus 111, the controller 107, and the needle controller 109 are explained.

First, the first feeder 117a is filled with the cell suspension 114. In case of the adhesive cells, the cells are separated from the carrier by trypsinization.

The mixing unit 118a mixes the cell suspension 114 that fills the first feeder 117a and the culture medium 115 that fills the second feeder 117b, and dilutes the cell suspension 114 to have the appropriate concentration to inject the transfer substance into the cell separately.

Then, the cell suspension is delivered to the apparatus 100 via the pipe 102a. Then the cell suspension enters the flow path 105. The flow path 105 is formed of a transparent material so that the apparatus 112 can observe the flow path 105.

The apparatus 112 observes the flow path 105 constantly. When the apparatus 112 detects the cell 121 that is delivered in the field of view, the apparatus 112 sends a cell-detection signal to the host computer 113. When the host computer 113 receives the cell-detection signal, the host computer 113 sends the trapping start command to the controller 107.

In compliance with the trapping start command from the host computer 113, the controller 107 operates the apparatus 106 to trap the cell 121 at a trapping hole 124 in the flow path 105, as shown in Fig. 9.

The apparatus 112 observes the flow path 105 constantly and when the apparatus 112 detects the cell that stops around the apparatus 106, the apparatus 112 sends a cell-trap competition signal to the host computer 113. The host computer 113, upon receiving the cell-trap competition signal, sends an injection start command, to start the injection of the gene, to the needle controller 109.

In compliance with the injection start command from the host computer 113, the needle driver 109 moves the needle 108 so that the needle 108 runs through the inserting hole 123 and sticks in the cell membrane of the cell 121 that has been trapped by the apparatus 106. Consequently, the needle 108 injects the transfer substance into the cell 121.

The transfer substance may be inserted in a hole in the needle 108, or be adhered to the tip of the needle 108. If the transfer substance is inserted in the needle 108, a liquid feeder of the needle controller 109 provides a control so that an appropriate amount of the transfer substance is injected in the cell.

When the needle controller 109 completes the operation of the gene transfer, the needle controller 109 sends a transfer competition signal to the host computer 113. In compliance with the transfer competition signal, the host computer 113 sends a release signal to the image processing apparatus 111 and the controller 107. Then, the image processing apparatus 111 terminates the operation of the signal detection, and the controller 107 operates the apparatus 106 to release the cell 121 that is trapped.

After the transfer substance is injected into the cell 121, the cell 121 is delivered to the second liquid feeder 1 03b via the flow path 105 and the pipe 102b. In the mixing unit 118b of the second liquid feeder 103b, the cell suspension that includes the cells 121 is mixed with the culture medium 115 and diluted to the appropriate concentration to treat the cells 121 separately.

The dispenser 104 dispenses the cell suspension, which is delivered from the second liquid feeder 103b and is diluted, to the general-purpose multi-hole plate. In this manner, the cells can be accommodated while permitting the separate cell operations.

After the cells 121 are accommodated separately, whether the cloning of the cell 121 appears and whether the effect of the gene transfer or the drug transfer appears can be confirmed as required.

It is mentioned in the previous explanation that, the cell 121 is trapped using the apparatus 106 shown in Fig. 9. However, the cell 121 may be trapped using a trapping device mechanically, or using any cytophilic substances.

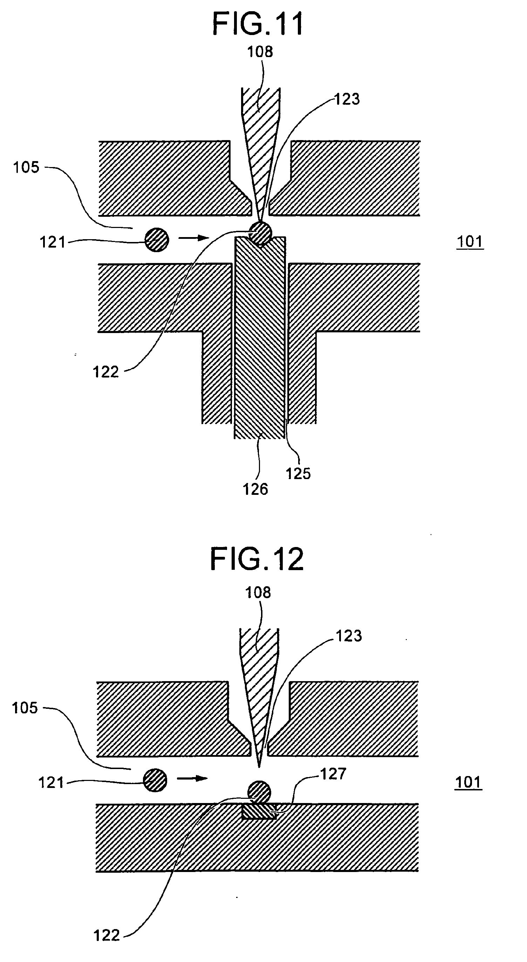

Moreover, as shown in Fig. 11, the cell 121 may be trapped using a trapping device 126. The trapping device 126 is inserted from an opening 125 and moved to the direction of an arrow to trap the cell 121 that is coming on the flow path 105. The transfer substance is injected into the trapped cell 122 using the needle 108 that is inserted from a micro hole 123 that is arranged in the wall surface of the flow path 105.

Furthermore, as shown in Fig. 12, the cell 121 may be trapped using a cytophilic substance 127. The cell 121 is adhered to the cytophlic substance 127 and gets trapped. The transfer substance is injected into the trapped cell 122 using the needle 108 that is inserted from the micro hole 123 that is arranged in the wall surface of the flow path 105.

When the tubes of the peripherals are connected to the body of the apparatus for injecting a substance into a cell, the tubes are connected to the body directly using the screw construction that is arranged on the end of the tube in the described embodiments. However, instead of connecting the tube and the body directly, a jig may be prepared on the apparatus so that the screw on the tube and the jig are connected.

Moreover, the connection is not limited to the screw construction. For example, the detachable component may be elasticized and fit to the opening using the elasticity.

Moreover, although all processes are automated using the image processing apparatus and the controllers in the above embodiments, some processes may be performed manually.

Moreover, although the apparatus for injecting a substance into a cell is formed of polycarbonate in the above embodiments, the apparatus for injecting a substance into a cell may be formed of another transparent resin, or glass.

Moreover, although the upper plate and the bottom plate that compose the apparatus for injecting a substance into a cell are explained as a single-layer structure, the multilayer structure may be employed according to the depth of holes and the like.

Moreover, although the flow path is arranged on the bottom plate, the flow path may be arranged on the upper plate with the cell input part and the cell output part.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

## Claims

1. An apparatus for injecting a substance into a cell, comprising:
a cell input part to input the cell;
a cell output part to output the cell;
a flow path that connects the cell input part and the cell output part, wherein the cell flows in the flow path from the cell input part toward the cell output part;
a trapping part that is arranged in the flow path and traps the cell that flows in the flow path; and
an inserting part that allows to pass a needle that injects the substance into the cell that is trapped at the trapping part, wherein
the cell injected with the substance flows in the flow path toward the cell output part and the cell can be retrieved from the cell output part.

2. The apparatus according to claim 1, wherein
at least one of the trapping part and the inserting part is formed of a transparent material.

3. The apparatus according to claim 1, wherein
at least one of the cell input part, the cell output part, and the trapping part includes an arrangement so that an external pipe can be detachably connected.

4. The apparatus according to claim 1, wherein
the cell input part, the cell output part, the flow path, and the trapping part are formed in a plate,
the flow path includes a bottom surface,
the trapping part includes a trapping opening and a trapping hole at a bottom surface of the trapping opening, wherein the trapping hole connects to the flow path,
the trapping hole has a diameter that is smaller than a diameter of the cell, and
a suction is generated at the trapping opening by an apparatus for trapping a cell at the trapping part.

5. The apparatus according to claim 4, wherein
the flow path includes a first part that is around the trapping part and a second part that is other than the first part, the first part is wider than the second part.

6. The apparatus according to claim 4, wherein
the flow path includes a first part that is around the trapping part and a second part that is other than the first part, the first part is meandering and the second part is substantially straight.

7. The apparatus according to claim 4, wherein
the bottom surface of the flow path include a step at the trapping part.

8. The apparatus according to claim 4, wherein
the inserting part is formed in another plate, which is different from the plate in which the cell input part, the cell output part, the flow path, and the trapping part are formed,
the flow path includes an upper surface,
the inserting part includes an inserting opening and an inserting hole at a bottom surface of the inserting opening,
the inserting hole has a diameter that allows insertion of the needle,
the inserting part is arranged opposed to the trapping part so that the flow path and the inserting opening are connected.

9. The apparatus according to claim 1, wherein the substance is any one of a gene solution and a drug solution.

10. The apparatus according to claim 1, wherein
the cell input part, the cell output part, the flow path, and the trapping part are formed in a plate,
the flow path includes a bottom surface,
the trapping part has a trapping hole arranged in the bottom surface of the flow path,
the trapping hole has a diameter that is smaller than a diameter of the cell, and
a suction is generated at the trapping hole.

11. The apparatus according to claim 1, wherein
the cell input part, the cell output part, the flow path, and the trapping part are formed in a plate,
the flow path includes a bottom surface,
the trapping part includes a trapping opening that connects to the flow path and a trapping rod capable of reciprocating in the trapping opening, wherein the trapping rod has a notch at an end to trap the cell.

12. The apparatus according to claim 1, wherein
the cell input part, the cell output part, the flow path, and the trapping part are formed in a plate,
the flow path includes a bottom surface,
the trapping part includes a substance having an adhesive power and arranged at the bottom surface of the flow path, wherein the cell gets stuck to the substance.

13. A system for injecting a substance into a cell, comprising:
an apparatus for injecting a substance into a cell, including
a cell input part to input the cell;
a cell output part to output the cell;
a flow path that connects the cell input part and the cell output part, wherein the cell flows in the flow path from the cell input part toward the cell output part;
a trapping part that is arranged in the flow path and traps the cell that flows in the flow path; and
an inserting part that allows to pass a needle that injects the substance into the cell that is trapped at the trapping part, wherein
the cell injected with the substance flows in the flow path toward the cell output part and the cell can be retrieved from the cell output part;
a liquid feeder that is connected to the cell input part;
a cultivation apparatus that is connected to the cell output part;
an apparatus for trapping a cell that is connected to the trapping part;
a needle driver that drives the needle; and
an observing part and a light source, whereby an operator can observe the cell trapped at the trapping part.

14. The system according to claim 13, wherein the observing part includes
a stage that supports the apparatus for injecting a substance into a cell; and
a limb that that supports a lens unit at least 30 millimeters above the apparatus for injecting a substance into a cell in such a manner that the lens unit can be rotated around the cell trapped at the trapping part at a constant distance, wherein
an operator can observe the needle that is inserted from the inserting part and the cell that is trapped at the trapping part simultaneously by positioning the lens unit at a desirable position.

15. A system for injecting a substance into a cell, comprising:
an apparatus for injecting a substance into a cell, including
a cell input part to input the cell;
a cell output part to output the cell;
a flow path that connects the cell input part and the cell output part, wherein the cell flows in the flow path from the cell input part toward the cell output part;
a trapping part that is arranged in the flow path and traps the cell that flows in the flow path; and
an inserting part that allows to pass a needle that injects the substance into the cell that is trapped at the trapping part, wherein
the cell injected with the substance flows in the flow path toward the cell output part and the cell can be retrieved from the cell output part;
a first liquid feeder that feeds a liquid that contains the cell to the cell input part;
a second liquid feeder that retrieves the cell from the cell output part; and
a dispenser that receives the cell retrieved by the second liquid feeder.

16. The system according to claim 15, wherein the first liquid feeder includes
a first feeder that feeds the cell suspension,
a second feeder that feeds culture medium, and
a mixing unit that mixes the cell suspension fed by the first feeder and the culture medium fed by the second feeder to produce the liquid; and
a liquid feeder controller that controls concentration of the cell suspension, amount of the culture medium that is fed, and liquid pressure of the culture medium so as to control a concentration of the liquid that is output from the mixing unit.

17. The system according to claim 15, further comprising:
a controller that controls the trapping part and movement of the needle, and
a cell observing unit that observes the cell that flows in the flow path, wherein
the cell observing unit sends a first signal to the controller upon the cell observing unit detecting the cell near the trapping part,
the controller sends a second signal to the trapping part upon receiving the first signal,
the trapping part starts a trap of the cell upon receiving the second signal,
the cell observing unit sends a third signal to the controller upon detecting that the trapping part has trapped the cell,
the controller makes the needle move and inject any one of a gene solution or a drug solution to the cell upon receiving the third signal,
the cell observing unit sends a fifth signal to the controller upon detecting that injection by the needle has completed,
the controller sends a sixth signal to the trapping part upon receiving the fifth signal, and
the trapping part releases the cell upon receiving the sixth signal.
